# EUROPEAN PATENT SPECIFICATION

(11) **EP 4 061 472 B1**
(45) Date of publication and mention of the grant of the patent: **24.07.2024**
(21) Application number: 20888824.8
(22) Date of filing: 19.11.2020
(51) Int. Cl.: A61N 1/04, A61N 1/36

(54) **AURICULAR NEUROSTIMULATION DEVICE AND SYSTEM, AND METHOD OF CONFIGURATION OF SUCH A SYSTEM**
VORRICHTUNG UND SYSTEM FÜR DIE NEUSTIMULATION DER OHREN, UND VERFAHREN ZUR KONFIGURATION EINES SOLCHEN SYSTEMS
DISPOSITIF ET SYSTÈME DE NEUROSTIMULATION AURICULAIRE, ET MÉTHODE DE CONFIGURATION D'UN TEL SYSTÈME

(30) Priority: 20.11.2019 EP 19383020
(43) Date of publication of application: 28.09.2022
(73) Proprietor: XanaStim Sàrl, 1066 Epalinges (CH)
(72) Inventor: LARRAYA, Iñaki, 31600 Burlada - Navarra (ES); LOPEZ FERNANDEZ, Miguel, 33391 Gijón - Asturias (ES); BERMEJO, Pedro Emilio, 28914 Leganés - Madrid (ES); GARCIA DE GURTUBAY, Iñaki, 31190 Cizur Menor - Navarra (ES); FARRE, Alberto, 25660 Alcoletge - Lérida (ES)
(74) Representative: Hernandez Lehmann, Aurelio
(86) International application number: PCT/EP2020/082688
(87) International publication number: WO 2021/099466

(56) References cited:
- WO-A1-2019/005774
- WO-A1-2019/014250
- WO-A1-2019/028000
- CN-A- 109 718 470
- DE-A1- 102015 012 684
- DE-B3- 102015 007 215
- US-A1- 2012 035 680
- US-A1- 2015 018 925
- US-A1- 2015 165 195

## Description

### Field of Invention

The present invention relates to a connected auricular neurostimulation device. The invention further refers to an auricular neurostimulation system comprising an auricular neurostimulation device having a higher efficiency and able to be personalized, adapted to each user and to each user's requirements. The invention further relates to a method of configuration of such a system.

### Background Prior Art

The vagus nerve (VN) is the longest cranial nerve and is involved in the regulation of multiple systems and maintaining homeostasis. As a slow-acting therapy, cervical vagus nerve stimulation (VNS) has been approved by the US Food and Drug Administration for managing treatment refractory epilepsy in 1997 and for chronic treatment-resistant depression in 2005. However, surgical risks and potential side effects have limited its application. To overcome such barriers, some non-invasive transcutaneous vagus nerve stimulation (taVNS) methods have been developed, superficially stimulating the cervical VN at the neck or at the outer ear.

The rationale of taVNS on the ear is based on anatomical studies demonstrating that certain parts of the ear area have afferent VN distribution, throughout the auricular branch of the vagus nerve (ABVN) and electrical stimulation of these areas may produce activity changes in the VN pathway in the brain stem and central structures, producing a modulation effect similar to invasive VNS.

The vagus nerve regulates metabolic homeostasis by controlling heart rate, gastrointestinal motility and secretion, pancreatic endocrine and exocrine secretion, hepatic glucose production, and other visceral functions. In addition, the vagus nerve is a major constituent of a neural reflex mechanism-the inflammatory reflex-that controls innate immune responses and inflammation during pathogen invasion and tissue injury.

TaVNS has been used to treat disorders, such as epilepsy, pre-diabetes, depression, chronic tinnitus, migraine, rehabilitation after ischemic stroke, ventricular arrhythmias, respiratory symptoms associated to COVID-19 as well as to boost associative memory what has been proposed to help patients with Alzheimer's disease and other dementia types. However, VNS has shown benefits beyond its original therapeutic application.

### Vagus nerve stimulation. Overview of neuroanatomical network.

The therapeutic mechanism of VNS (both invasive and taVNS) is thought to be mediated by concentration shifts of noradrenaline, γ-aminobutyric acid (GABA) and acetylcholine (ACh) in the central nervous system, which induce neuroplastic changes in the cerebral cortex.

The nucleus of the solitary tract, or nucleus tractus solitarius (NTS), is the recipient of most afferent sensory fibers, but the vagus also sends ipsilateral projections to the area postrema, dorsal motor nucleus of the vagus, nucleus ambiguus, medullary reticular formation, and the spinal trigeminal nucleus. The NTS is an important processing and relay center for a variety of vital functions, so in addition to these vagal projections, it also integrates inputs from the glossopharyngeal, facial, and trigeminal nerves, and numerous brain regions. The NTS, sends monosynaptic projections to diffuse regions of the brain, such as the facial, trigeminal, and hypoglossal nuclei, the dorsal motor nucleus of the vagus, nucleus ambiguous, the parabrachial nucleus, pons, and the respiratory and cardiovascular centers located on the ventral surface of the medulla. Additionally, monoamine nuclei in the brainstem, the locus coeruleus (LC) and the raphe nuclei, receive direct and/or indirect projections from the NTS. Forebrain and limbic structures also receive NTS projections, including the bed nucleus of the stria terminalis, paraventricular, dorsomedial, and arcuate hypothalamic nuclei, preoptic and periventricular thalamic nuclei, and central amygdaloid nucleus. After VNS, even the brain cortex is affected where an increased GABA neurotransmitter concentration has been described.

Since the activity of a great number of structures is hacked after stimulating the vagus nerve, a great number of body and brain functions change and some of them could increase physical and cognitive performance. Although these changes are many, they can be categorized in four different groups: recovery, improvement of cognitive and motor skills, stress control and body weight and composition control.

### Recovery.

One beneficial mechanism behind exercise is the reduction of inflammation when performed regularly. Clinical studies show that consistent exercise reduces some inflammatory cytokines and can promote health for this reason. On the other hand, little or no exercise promotes increased inflammation. However, high levels of exercise can also promote high levels of inflammation and affect recovery. This equilibrium between pro-inflammatory and anti-inflammatory factors is extremely important for example for elite athletes.

Inflammation is normally a local and temporary event, and after its resolution, immune and physiological homeostasis is restored. This response is especially important for some sports. For example, weightlifters depend upon some inflammation to break down muscle when they lift. Then they take a break to allow the regrowth of the muscle, and it comes in stronger and bigger. Therefore, it is important to take some days off after heavy lifting or to alternate body parts being trained, to allow the inflammation to normalize. Otherwise, further exercise and inflammation does not allow this normal recovery and can ultimately result in damage to the muscle and the inflammation can begin to cause problems systemically.

Acute inflammation allows strengthening, but chronic inflammation is damaging. This inflammation has been described in most but especially most demanding sports and in elite athletes. In triathletes, a study described extremely large increases in creatine kinase, C-reactive protein, aldosterone and cortisol combined with reductions in testosterone and the testosterone:cortisol ratio. Another study evaluated the immediately post-race parameters in these subjects and demonstrated significant increases in total leukocyte counts, myeloperoxidase, polymorphonuclear elastase, cortisol, creatine kinase activity, myoglobin, IL-6, IL-10 and high-sensitive C-reactive protein, while testosterone significantly decreased compared to prerace. Another study demonstrated that exhaustive exercise induces systemic inflammatory responses, which are associated with exercise-induced tissue/organ damage. Particularly, as the activation of the master regulatory factor nuclear factor (erythroid-derived 2)-like 2 (Nrf2) is involved directly or indirectly in controlling pro-inflammatory genes and antioxidant enzymes expression, whilst nuclear factor-kappa B (NF-κB) regulates the pro-inflammatory gene expression.

This inflammatory component lasts even for one week or even more, and only after this period, athletes could continue their full training. However, this period of recovery is too much for most elite athletes due to the type of competition (cyclists), to a high number of matches in the championship period (baseball, soccer, basketball) or just because they need to train more often to improve their results. Therefore, the development of strategies to decrease this recovery period could be extremely useful for these professionals and VNS could play a role in shortening this time.

This chronic proinflammatory state in athletes has been widely studied in recent years. Proinflammatory cytokines, along with chemokines, reactive oxygen species, nitrogen intermediates and other inflammatory molecules, are critically implicated in extracellular pathogen clearance, vasodilatation, neutrophil recruitment, increased vascular permeability and induction of acute-phase proteins, such as C-reactive protein and coagulation molecules. This proinflammatory progression is usually balanced by the release of IL-10, TGF-β, soluble cytokine receptors and other anti-inflammatory molecules, but when exercise is frequent and intense, as usually occurs in elite players, the proinflammatory cascade predominates and the systemic and chronic inflammation persists, possible reflecting incomplete muscle recovery.

This disrupted immune regulation can result in continual proinflammatory cytokine activity and excessive or chronic inflammation. This state not only could prevent recovery in professional athletes but could be associated with a range of disease syndromes, including sepsis, rheumatoid arthritis, inflammatory bowel disease and other inflammatory and autoimmune disorders. The vagus nerve could help to control inflammatory cells and the release of inflammatory cytokines when inflammation is no longer needed.

The body's adaptation to physical exercise is modulated by sympathetic and parasympathetic (vagal) branches of the autonomic nervous system (ANS) and it is usually measured by heart rate variability (HRV), the beat-to-beat variation of the heart. Although vagal activity is usually stimulated after exercise, after terminating strenuous exercise in professional athletes the vagal activity is impaired and autonomic nervous regulation seems to be postponed which is reflected in reduced HRV, whereas the early recovery of the vasculature, post-exercise hypotension, is still preserved. This means the autonomic nervous system regulation is impaired in professional players and the sympathetic branch of the ANS predominates, which could be associated with a long term pro-inflammatory state and its related complications. Another study evaluated how long it takes to appear this sympathetic dominance in athletes and established that a shift towards relative sympathetic dominance, particularly due to reduced vagal activity, was apparent after approximately 8 years of competing at the professional level.

Some authors establish the relationship between this sympathetic predominance and some health problems frequently observed in athletes. Aubert, et al, 2001 concluded that HRV is affected by chronic exercise, especially in endurance trained athletes and infers that especially aerobic exercising can have beneficial effects on the cardiovascular risk profile. However, although regular physical exercise clearly reduces cardiovascular morbidity risk, long-term endurance sports practice has been recognized as a risk factor for atrial fibrillation (AF). On the other hand, Cole et al, 1999, considered decreased vagal activity, evaluated by a delayed decrease in the heart rate during the first minute after graded exercise, as a powerful predictor of overall mortality, independent of workload, and established a relationship with decreased vagal tone and the presence of myocardial perfusion defects and changes in heart rate during exercise.

VNS is a novel strategy that has demonstrated efficacy to treat inflammatory conditions and has been hypothesized to prevent the chronic pro-inflammatory condition in elite athletes. In fact, VNS increases levels of the anti-inflammatory cytokine IL-10 and decreases others such as the pro-inflammatory cytokines such as TNF-α, IL-1β, and IL-6. These specific pro-inflammatory cytokines, IL-6, IL-8 and TNF-α are significantly elevated in all professional soccer players.

In the recent years, more and more studies have demonstrated that vagal activity is inversely related to chronic inflammation, raising the possibility that vagal regulation of immune reactivity may represent a pathway linking psychosocial factors to risk for inflammatory disease, independently of demographic and health characteristics, including age, gender, race, years of education, smoking, hypertension, and white blood cell count. An implantable vagus nerve-stimulating device in epilepsy patients has also been demonstrated to inhibit peripheral blood production of TNF-α.

Therapeutic VNS activates both efferent and afferent fibers of the vagus nerve; however, the effects attributable to vagal afferent stimulation are unclear. It seems to be mediated through several pathways, although some of them are still debated. The first pathway is the anti-inflammatory hypothalamic-pituitary-adrenal axis which is stimulated by vagal afferent fibers and leads to a decrease of cortisol. The second one, called the cholinergic anti-inflammatory pathway, is mediated through vagal efferent fibers that synapse onto enteric neurons which release acetylcholine (ACh) at the synaptic junction with macrophages. ACh binds to α-7-nicotinic ACh receptors (α7nAChR) of those macrophages to inhibit the release of TNF-α. The last pathway is the splenic sympathetic anti-inflammatory pathway, where the VN stimulates the splenic sympathetic nerve. Norepinephrine (noradrenaline) released at the distal end of the splenic nerve links to the β2 adrenergic receptor of splenic lymphocytes that release ACh. Finally, ACh inhibits the release of TNFα by spleen macrophages through α-7-nicotinic ACh receptors.VN stimulation, either as an invasive or non-invasive procedure, is becoming increasingly frequent and several clinical trials are ongoing to evaluate the potential effectiveness of this therapy to alleviate chronic inflammation. In fact, this provides a new range of potential therapeutic approaches for controlling inflammatory responses.

### Improvement of cognitive and motor skills.

Cognitive and motor skills are important in everyday living for everybody. Improving attention, concentration, memory, reaction time or specific motor skills could be a competitive advantage in some activities.

VNS has been associated with an increase of certain neurotrophins, particularly brain-derived neurotrophic factor (BDNF) and basic fibroblast growth factor (bFGF), that could influence neurogenesis in the adult rat hippocampus, increasing memory. BDNF might have a role in the protection mechanism against brain damage and contributes in occurrence and maintenance of high attention and concentration especially among combat sports. BDNF binding to its receptor, TrkB and VNS is known to stimulate not only the production of BDNF but its receptor, TrkB, increasing its action.

VNS is known to increase norepinephrine (NE) in the brain. NE is thought to improve several aspects of cognitive control, including the suppression of irrelevant information that could help to focus and concentrate. Suppressing irrelevant information in decision making is an essential everyday skill, over all in certain sports such as golf, baseball, basketball, soccer, or combat. A study demonstrated that VNS improved ability to suppress distractor interference and increase cognitive control. Another study demonstrated that VNS improved working memory performance, reduced errors on subtasks that relied on working memory and increased reaction time in response to distractors. A recent study suggested that chronic VNS could be used to improve some learning processes, such as foreign languages.

In fact, chronic VNS improves attention and concentration and avoids distractions not only in healthy subjects, but also in some neurological disorders such as refractory depression. When VNS was applied, sustained clinical and cognitive improvements were seen in these patients, with some mental functions improving as soon as one month after the initiation of the VNS therapy.

Creativity is one of the most important cognitive skills in our complex and fast-changing world. Previous evidence showed that GABA is involved in divergent but not convergent thinking. Results demonstrate active taVNS, compared to sham stimulation, enhanced divergent thinking, and what is associated with creativity. A study suggested that GABA (likely by taVNS) supports the ability to select among competing options in high selection demand (divergent thinking) but not in low selection demand (convergent thinking) which could also be crucial for professional athletes. Another study demonstrated that taVNS enhances response selection processes when selection demands are particularly high.

But VNS could not only improve cognitive but also motor skills. Improving motor skills has been associated with motor cortex plasticity. In fact, this motor cortex plasticity is related with the ability to acquire new skills and adaptations. A higher motor plasticity is thought to enhance motor learning.

As VNS increases this plasticity in the motor cortex, it could help to improve these motor skills. Although there is no evidence about the possibility that VNS could stimulate plasticity in the motor cortex and improve motor skills, there is a lot of experience in improving motor plasticity in brain damage patients. VNS paired with rehabilitative interventions improves motor recovery in chronic stroke. For these patients, noninvasive approaches, such as taVNS are safe, well-tolerated and improves motor function in those with residual weakness. Another study demonstrated that VNS paired with rehabilitative therapy improved motor results in traumatic brain injury, compared with the placebo group.

### Stress control.

Psychological stress and recovery monitoring are key issues for health, well-being, and performance. People are frequently exposed to various situations and conditions that can provoke chronic stress and interfere with their normal performance. These stressful situations affect autonomic nervous system activity and hormonal responses. In fact, lellamo, et al, 2003, indicated in their study a dissociation of the neural and hypothalamic-pituitary-adrenal axis functioning in response to the stress of competition in elite athletes, and the considerable extent to which competition may alter selectively the physiology of stress-related hormones while sparing autonomic cardiac regulation and affecting their productivity and performance.

Chronic stress is also associated with sleep deprivation. Nedelec, et al, 2015 demonstrated in a study that sleep deprivation in elite soccer players may be detrimental to the outcome of the recovery process after a match, resulting in impaired muscle glycogen repletion, impaired muscle damage repair, alterations in cognitive function and an increase in mental fatigue, what can be another reason to treat chronic stress and this way increase recovery and performance.

Chronic stress has been associated with a decreased vagal tone and an increase in reaction time and decision-making impairment. Some athletes work exhaustive schedules and are under important psychological pressure which could decrease vagal nerve tone and impair decision making processes and increase reaction time which could finally impair their physical and cognitive performance. A chronic vagal nerve stimulation could reverse this decrease in vagal tone and therefore, reverse these performance impairments.

This relationship between vagal tone decrease and chronic stress has also been documented for other situations. Zanstra et al, 2006 studied differences in task performance and related sympathetic-vagal reaction patterns between burnouts and controls during a mentally demanding workday and suggested the burnout group had a sympathetic predominance in the sympathetic-vagal balance. Burnouts experienced an increase in effort and were more tired at the end of the workday.

TaVNS both in isolation and following exposure to stress reduces sympathetic and enhances parasympathetic function, leading to a modulation in autonomic tone, which could be used to reduce stress not only in elite athletes but in a good percentage of the population in current societies. This modulation in autonomic tone can be evaluated by spectral analysis of heart rate variability (HRV), which is a simple, non-invasive technique that is widely used to assess sympatho-vagal regulation of the heart. Its employment is increasing partly due to the current rising usage of wearable devices. During chronic stress, the sympathetic nervous system is hyperactivated, causing physical, psychological, and behavioral abnormalities. The current neurobiological evidence suggests that HRV is impacted by stress and supports its use for the objective assessment of psychological health and stress. Heightened occupational stress was found associated with lowered HRV, specifically with reduced parasympathetic activation. Some studies describe autonomic dysregulation using HRV, and these autonomic alterations are related to level of performance. The measurement of HRV is often considered a convenient non-invasive assessment tool for monitoring individual adaptation to training. Decreases and increases in vagal-derived indices of HRV have been suggested to indicate negative and positive adaptations, respectively, to endurance training regimens.

HRV can be used to measure an athlete's adaptation to training load, without disrupting the training process. More and more studies in the recent years have demonstrated that sympathetic dominance, considered as a sign of physical or mental fatigue and chronic stress, could be harmful for athletes' performance and, this way, VNS could reverse this abnormal dominance. In fact, maintaining high vagal activity during the preseason has also been associated with better results. The cardiac autonomic imbalance observed in overtrained athletes implies changes in HRV and therefore would consider that heart rate variability may provide useful information in detection of overtraining in athletes and can be a valuable adjacent tool for optimizing athlete's training program. On the other hand, heart rate recovery (HRR) early after exercise is dependent primarily on parasympathetic reactivation. Thus, accelerated HRR early after exercise in endurance-trained athletes may be attributed to augmented parasympathetic reactivation.

Other biomarkers related to chronic have also been modified by taVNS demonstrating its role in treating this condition. Salivary alpha amylase and cortisol are modified using taVNS compared to sham which supports the use of taVNS to treat chronic stress.

### Body weight and composition.

Recently vagus nerve has been involved in weight control, and muscle preservation. The vagus nerve innervating the gut plays an important role in controlling metabolism. It communicates peripheral information about the volume and type of nutrients between the gut and the brain. Depending on the nutritional status, vagal afferent neurons express two different neurochemical phenotypes that can inhibit or stimulate food intake. Chronic ingestion of calorie-rich diets reduces sensitivity of vagal afferent neurons to peripheral signals and their constitutive expression of orexigenic receptors and neuropeptides. This disruption of vagal afferent signaling is sufficient to drive hyperphagia and obesity. Furthermore, neuromodulation of the vagus nerve can be used in the treatment of obesity. Although the mechanisms are poorly understood, vagal nerve stimulation prevents weight gain in response to a high-fat diet. In small clinical studies, in patients with depression or epilepsy, vagal nerve stimulation has been demonstrated to promote weight loss, and vagus nerve dysfunction has been associated with higher body mass index. In conclusion there is strong evidence that the vagus nerve is involved in the development of obesity and it is proving to be an attractive target for the treatment of obesity.

Other studies in animal models have highlighted the importance of vagus nerve in affecting body mass. In pigs, VNS attenuated body weight gain and backfat gain resulting in lower back fat depth/loin muscle ratio. In rats, VNS can regulate food intake in obese animals. These works correlate nerve stimulation with highly effective weight control. Although reasons for weight loss are unknown, the reduction in body fat induced by VNS in rats may result from the action of both central and peripheral mediators. The reduced feed conversion efficiency associated with VNS may be mediated by hypothalamic BDNF, down-regulation of endocannabinoid tone in mesenteric adipose tissue and a PPARα-dependent increase in fatty acid oxidation in the liver, which in concerted action may account for the anorexic effect and increased energy expenditure.

However, vagus nerve is not only related to body weight but its composition, modulating the percentage of fat, another interesting parameter for athletes. In fact, sympatho-vagal imbalance seems to be associated with sarcopenia in male patients. This idea has suggested that VNS could be a therapeutic approach for patients with muscle wasting and increased peripheral sympathetic outflow. VNS significantly reduced cellular apoptosis, necrosis, and inflammatory cell infiltration compared to sham VNS. The VNS treatment also decreased the inflammatory response, alleviated oxidative stress, and improved vascular endothelial function.

Skeletal muscle produces and releases significant levels of IL-6 after prolonged exercise and is therefore considered as a myokine. On the other hand, muscle is also an important target of the cytokine. IL-6 signaling has been associated with stimulation of hypertrophic muscle growth and myogenesis through regulation of the proliferative capacity of muscle stem cells. Additional beneficial effects of IL-6 include regulation of energy metabolism, which is related to the capacity of actively contracting muscle to synthesize and release IL-6. Paradoxically, deleterious actions for IL-6 have also been proposed, such as promotion of atrophy and muscle wasting. Some inflammatory cytokines such as IL-6, COX-2 and uPA may play roles in the inhibition of skeletal muscle growth induced by overtraining, something frequently seen in elite athletes, and could be reversed by VNS.

Additionally, muscle regeneration and growth are greatly slowed by loss of IL-10. IL-10 plays a central role in regulating the switch of muscle macrophages from a M1 to M2 phenotype in injured muscle in vivo, and this transition is necessary for normal growth and regeneration of muscle. VNS has also demonstrated to increase IL-10 levels and therefore, participate in muscle regeneration and growth.

VN is also associated with the release of some hormones related to muscle growth or loss. On the one hand, it is thought that testosterone secretion is regulated by the vagus nerve. A study with animals demonstrated that testosterone concentration decreased significantly in right vagotomized rats, and different responses to testosterone have been associated with different vagal responses. Therefore, although more studies are warranted, VNS could help athletes increase their muscle growth. On the other hand, VN is related to the secretion of ghreline, a hormone related to multiple mechanisms such as cognition, learning and memory, the sleep-wake cycle, taste sensation, reward behavior, and glucose metabolism. This hormone is also related to secretion of growth hormone, a substance clearly related to muscle growth. However, the relationship between VNS and the ghrelin and leptin equilibrium is complex and more studies are also warranted. The relationship between VNS and IGF-I concentration also needs to be evaluated since this hormone is a key factor for muscle growth and there is only one study suggesting IGF-1 lower plasma levels and VNS.

US 2012/0035680 (A1) and WO 2019/014250 (A1) describe a device that electrically stimulates afferent fibres of the auricular branch of the vagus nerve taking into account the user's pulmonary activity (Respiratory-gated Auricular Vagal Afferent Nerve Stimulation - RAVANS). The control of the stimulation is carried out using an electrical circuit connected on one side to two electrodes that apply the stimulation voltage and on the other side to a respiratory belt with a strain gage, a nasal air flow detector (US 2012/0035680 (A1)) or a pulse sensor configured to measure blood pressure (WO 2019/014250 (A1)) that send electrical signals associated with the pulmonary activity. The fact that the user has to carry and connect several devices significantly reduces the usability of the device.

The device incorporates two electrodes attached to afferent fiber zones of the auricular branch of the vagus nerve wherein the electrodes are described as *"small discs made from conductive material and attached to the patient using an adhesive band. Similarly, pre-gelled circular or spherical silver*/*silver chloride electrodes can be used".*

WO 2019/005774 (A1) describes a device for transcutaneous electrical stimulation of peripheral nerves including the auricular branch of the vagus nerve. The device comprises a control unit and a housing to be placed on or in the ear, with two electrodes connected to the control unit, which can modulate the electrical current applied to the electrodes. The control unit or the housing can be fitted with a sensor to measure the user's physiological parameters, on the basis of which the stimulation parameters can be adjusted. These parameters include heart rate variability (HRV) and oxygen saturation.

This document discloses a pair of electrodes that are located "on an external periphery of an cylindrical interface member having a C-shaped cross-section that engages a target portion of a patient's ear".

The device described uses therefore a standard geometry housing for the electrode holder, which does not ensure that the contact with the area to be stimulated is good enough due to the great anatomical variability of human ears. Besides, the disclosed device uses standard electrode geometry and due again to the anatomical variability this does not ensure that the contact is sufficiently wide and of good quality. Both of the above characteristics make the stimulation less effective and comfortable. On the other hand, the document does not mention whether the stimulation is anodic or cathodic. The addition of a delay between the stimulation pulse and the reversion pulse is also not mentioned.

EP3100764 (A1) shows a neurostimulation device from Cerbomed that stimulates the nerve ramifications only of cymba by means of two electrodes. However, these two electrodes are small as they must fit in the cymba reserving a space between them to avoid short circuit. On the other hand they are located on a standard support that has limitations to adapt to the variable geometry of the cymba of the users, reason why in many cases the contact of the electrodes with the cymba is very poor. Both characteristics make the stimulated area of the cymba very small and the general efficiency of the device is reduced. In addition, all the electronics of the device are external (outside the ear cavity), which implies significant needs for wiring, connections, etc. that make the device as a whole very bulky and uncomfortable to use.

PCT/EP2015/001279 discloses a stimulation pattern of a neurostimulator similar to that of EP3100764 (A1). It is a trapezoidal, asymmetric biphasic wave starting with a positive pulse (anodic stimulation). It is estimated that the depolarization that occurs with anodic stimulation is roughly one-seventh to one-third that of the depolarization with cathodic stimulation (the waveform begins with a negative pulse).

These known devices use electrical current as a means of stimulating nerve endings.

However, an anatomical study performed by the applicants found that nerve endings in the auricular region correspond to mechanoreceptors and thermoreceptors that respond to mechanical and thermal stimuli, respectively. Therefore, it is also possible to activate these nerve endings with mechanical stimuli such as fine touch or vibration, thus resulting in more efficient devices, easier to adapt to the users. Furthermore, known devices stimulate nerves in the ear in areas where there is no high concentration or no concentration at all of ABVN, thus resulting in inefficient devices. Moreover, personalization to adapt correctly these devices so they fit to each user is key, and is not provided by the devices known in the state of the art.

The object of the invention is a wearable connected auricular neurostimulation device that stimulates the nerve ramifications of cymba and cavum conchae having a higher efficiency, being comfortable to wear and allowing to be personalized such that it can adapt to each user and to each user's requirements.

The invention also aims at other objects and at the solution of other problems as will appear in the rest of the present description.

### Summary of the invention

The invention is defined in the device claim 1, the system claim 12, and the method claim 14. Further aspects and preferred embodiments are defined in the appended claims. Aspects, embodiments and examples of the present disclosure which do not fall under the scope of the appended claims do not form part of the invention and are merely provided for illustrative purposes.

In view of the foregoing prior art, an object of the present invention is to provide, according to a first aspect, an auricular neurostimulation device configured as an wireless earbud or auricular wearable by a user and configured to stimulate the Auricular Branch of Vagus Nerve (ABVN) on the user's ear; the device comprising at least one electrode designed to be located in the cymba and another electrode in the cavum conchae. The cymba electrode uses the entire area of the cymba to stimulate the ABVN present in the zone when a voltage difference is applied to it with respect to the cavum conchae electrode.

Preferably, the electrodes are made of graphene, biocompatible metals such as titanium, nickel titanium (nitinol), platinum, platinum-iridium, non-toxic metals as gold, conductive biocompatible inks for 3D printing or flexible conductive biocompatible polymers that adapt to the anatomy of the stimulation zone, therefore providing good comfort and perfect adaptation to the patient's ear.

Typically, the auricular neurostimulation device further comprises an earmold where the electrodes are arranged, the earmold being customized to the user's anatomy to achieve good contact between the electrodes and the stimulation zones.

Preferably, the auricular neurostimulation device of the invention further comprises a photoplethysmographic or biosensor estimating the amount of hemoglobin and oxyhemoglobin circulating through the most superficial capillary vessels of the ear of the patient or user, these data being used to calculate the heart rate, the heart rate variability (HRV) and to detect the breathing phase (exhalation or inhalation) of the user.

The device in this application also detects the phases of inspiration/expiration in order to be able to stimulate selectively during expiration, but this is done with the photoplethysmography technique that allows the measurement to be made on the ear itself. The latter allows us to integrate the stimulation circuit, the breathing phase detection device (sensor) and the controller in the same circuit that can be housed inside the ear's auricle.

Typically, the photoplethysmographic or biosensor is configured to detect a low heart rate of the user (bradycardia), in which case the stimulation of the device will be stopped to avoid any cardiological risk.

Preferably, in the auricular neurostimulation device of the invention, the stimulation done by it is synchronized with the exhalation-breathing phase of the user.

The auricular neurostimulation device of the invention typically implements three types of stimulation protocols: BEAT, BFS and EVANS, with variable stimulation parameters in all of them.

Preferably, the stimulation protocols are based on a waveform of rectangular, biphasic, symmetric and with a delay between the negative and positive pulse.

In one preferred embodiment, the stimulation protocol is of the BEAT type, consisting of the continuous application of bursts of pulses.

In another embodiment, the stimulation protocol is a BFS (Breathing Focused on Stimulation) type combining stimulation moments with standstill moments, so the user breathes in during the stop time and breathes out during the stimulation. This type of protocol makes it easier for the user to focus his attention on stimulation and thus benefit from the meditation effect. It also allows the duration of breathing to be extended and the benefits of slow breathing to be added. In this way, the protocol BFS adds to the beneficial effect of relaxation, meditation and slow breathing.

In another embodiment, the stimulation protocol is a EVANS (Exhalation Vagus Auricular Nerve Stimulation) type, in which the stimulation is also synchronized with the user's exhalation but without the user's attention being necessary, since the stimulator detects the respiratory cycle and only stimulates during exhalation. In this way, the user can devote his attention to other activities because the stimulator is responsible for stimulating in sync with the breath.

Preferably, the electrical charge applied to each user in each stimulation can be personalized. Initially, each user is assigned an electrical charge depending on his/her profile, but based on the analysis of the data captured by the biosensor, the electrical charge to be injected can be customized. The stimulator keeps track of the applied electrical charge by stopping the stimulation when the set electric charge of the session has been reached. It is also possible to assign a maximum daily dose that the device will control not to be exceeded.

According to a second object, the invention relates to an auricular neurostimulation system comprising an auricular neurostimulation device and a charging case where the device can charge an internal battery and where the device discharges into this case the data captured by the photoplethysmographic sensor during stimulation and sends them to a dedicated platform in the cloud.

Typically, the auricular neurostimulation system of the invention further comprises a smartphone application that allows the user to interact with the neurostimulator.

The cloud platform can also integrate data obtained from devices for continuous monitoring of cardiac activity such as watches, bracelets or rings, among others. The analysis of these data allows, for example, to know what the pattern of evolution of a user's stress is like and to define personalized stimulation treatments to prevent high peaks.

According to a third aspect, the invention relates to a method of configuration of an auricular neurostimulation system comprising the following steps:
Creation, via the smartphone application, by the user of a user account using the application for the smartphone or the web entering a series of personal data;
Assignment, via the smartphone application, of an initial electrical charge value to apply in each stimulation session and a maximum daily electrical charge, depending on the user's profile and on the basis of statistical studies;
Detection of the user logging into the application;
Connection to the auricular neurostimulation device to match a serial number of the auricular neurostimulation device to the user account, so the device is associated to the user;
Prompting the user to set his/her "perception and pain thresholds";
Based on both thresholds establishment of a range in which the stimulation intensity must be placed, so that the stimulation is effective but also comfortable;
Detection of a selection of a stimulation protocol by the user; and
Configure the auricular neurostimulation device in accordance with the range of stimulation intensity and the stimulation protocol.

### Brief description of the drawings

Further features, advantages and objects of the present invention will become apparent for a skilled person when reading the following detailed description of non-limiting embodiments of the present invention, when taken in conjunction with the appended drawings, in which:
**FIG. 1****:** Detailed view of the different ear areas that may be stimulated in a human person.
**FIG. 2****:** Perspective view of the auricular neurostimulation device according to a first preferred embodiment of the present invention, showing its main components.
**FIG. 3****:** Lateral perspective view of the auricular neurostimulation device according to a first preferred embodiment of the present invention, as represented in Figure 2.
**FIG. 4****:** Perspective view of the auricular neurostimulation device according to a first preferred embodiment of the present invention, as represented in Figure 2, shown in the position where it will be placed in the ear of the patient.
**FIG. 5****:** Perspective view of the auricular neurostimulation device according to a first preferred embodiment of the present invention, as represented in Figure 2, from its bottom position.
**FIG. 6****:** Scheme with the components of the connected auricular neurostimulation system of the invention.
**FIG. 7****:** Perspective view of the auricular neurostimulation device of the invention in an alternative mode of implementation to that in Figure 2, with the electronics located Behind The Ear (BTE).
**FIG. 8A**:Graph showing the stimulation pattern of the auricular neurostimulation device of the present invention.
**FIG. 8B**:Graph showing the stimulation pattern of the auricular neurostimulation device of the present invention, synchronized with the patient's exhalation.
**FIG. 9A**: A first exemplary layout of a Printed Circuit Board (PCB) of the auricular neurostimulation device of the invention, where discontinuous lines represent the flexible parts and the continuous lines the rigid parts.
**FIG. 9B****:** A second exemplary layout of a Printed Circuit Board (PCB).
**FIG. 10****:** Graph showing the Vagus sensory evoked potential (VSEP) induced by auricular stimulation applied comparing the auricular neurostimulation device of the present invention with a prior art stimulation device.
**FIG. 11****:** Landmarks and lengths to characterize the surface of the cymba.

### Detailed description of the exemplary embodiments

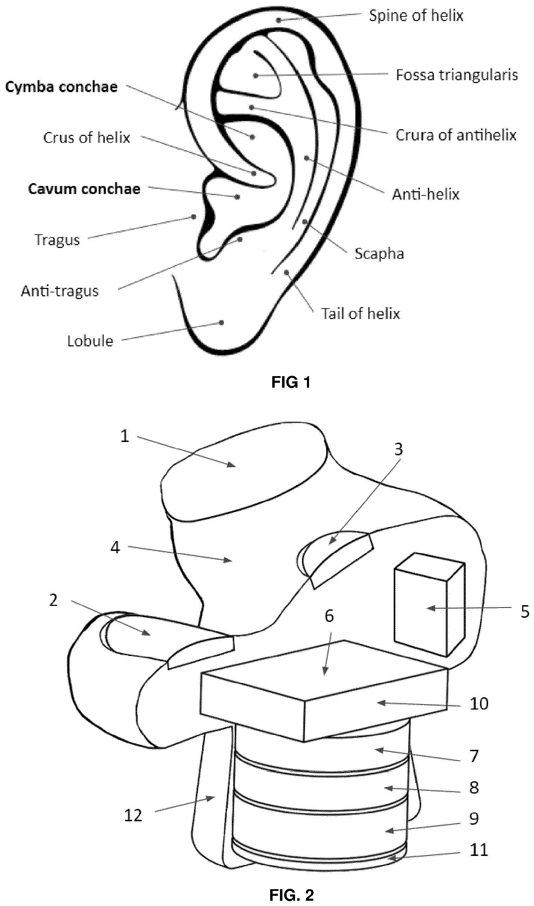

The object of the invention is a connected auricular neurostimulation device 1, wearable by a patient that optimizes the stimulation of the ABVN present in the cymba and cavum conchae, as it can be seen in Figure 1.

The auricular neurostimulation device 1 of the invention comprises the following components, as represented in Figure 2, according to a first preferred embodiment where the device 1 is arranged in the patient's ear:
- An electrode 2 that occupies the entire section of the cymba (the only ear zone with 100% ABVN). In a preferred embodiment, this electrode is configured as a working electrode on which a cathodic stimulation is applied in order to maximize the activation of the ABVN.
- An electrode 3 placed in the cavum conchae (ear zone with 45% ABVN). In a preferred embodiment, this electrode serves as a reference for applying the electrical voltage difference generated by cathodic stimulation.
- The electrodes are typically manufactured using graphene, biocompatible metals such as titanium, nickel titanium (nitinol), platinum, platinum-iridium, non-toxic metals as gold, conductive biocompatible inks for 3D printing or flexible conductive biocompatible polymers that adapt to the anatomy of the stimulation zone, therefore providing good comfort and perfect adaptation to the patient's ear.
- Earmold 4: This part is used as a support for the cymba and cavum conchae electrodes 2, 3 respectively in order to ensure that the positioning of these electrodes 2, 3 is adequate to maximize cymba and cavum conchae stimulation. The advantage of this earmold 4 in the device 1 of the invention is that it is custom made to the user's anatomy, so it can be personalized and shaped to optimally fit each patient's or user's anatomy. The earmold material is biocompatible and preferably thermoelastic so that it improves the fit with the user's ear as the earmold acquires body temperature.
- Photoplethysmographic or biosensor 5: This sensor serves to measure the environment temperature and the user's temperature and to estimate the amount of hemoglobin and oxyhemoglobin circulating through the most superficial capillary vessels of the ear of the patient or user, once the device 1 has been arranged on him or her. These data can be used to calculate the heart rate, the heart rate variability (HRV) and the oxygen saturation, among other biomedical variables and to detect the breathing phase (exhalation or inhalation) of the user or patient.

This photoplethysmographic or biosensor 5 is able to detect a very low heart rate of the user: in case this is detected, the device 1 is configured to automatically stop the stimulation.

In addition, the measurements made by the sensor make it possible to know how much electrical charge needs to be applied to each user at any given time to achieve vagal activation. This allows personalizing the stimulation treatments reaching efficiency levels much higher than other existing devices known in the art.

Moreover, the photoplethysmographic or biosensor 5 is also able to detect the breathing phase (exhalation or inhalation) of the patient or user wearing it with the aim to automatically synchronize the stimulation of the device 1 only with the user's exhalation with the goal to obtain a more efficient activation of the vagus nerve.
- Electronic circuit 6: The auricular neurostimulation device 1 is equipped with an electronic circuit 6 allowing it to develop the following functionalities, as indicated below.
   - Generation of stimulation patterns with variable duration, intensity, frequency of bursts and pulses, number of pulses per burst, pulse widths, and pulse delays, amongst others, by applying electrical voltage differences between electrodes 2 and 3.
   - Generation of stimulation patterns synchronized with the exhalation of the user.
   - Control of electrical charge applied in each stimulation and daily-accumulated charge.
   - Exchange of data with external devices through wireless connections.
   - Data exchange with a charging case.
   - Wireless charging of the battery 10 by electromagnetic induction with a charging case.
- Faceplate 12: The auricular neurostimulation device 1 is equipped with a faceplate 12 that protects the electronic circuit 6 and makes it easy for the user to pick the device up for its placement and removal in the user's ear and in the charging case 13.

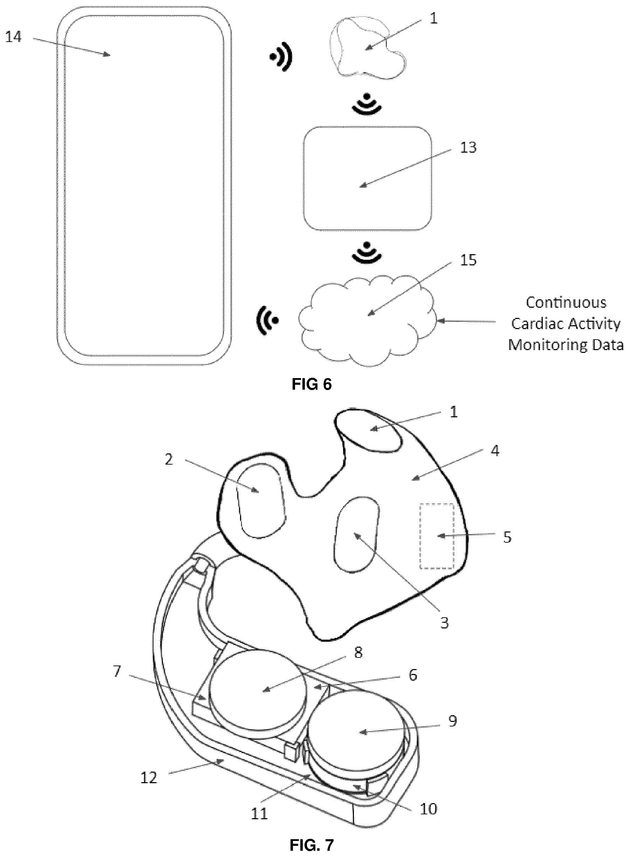

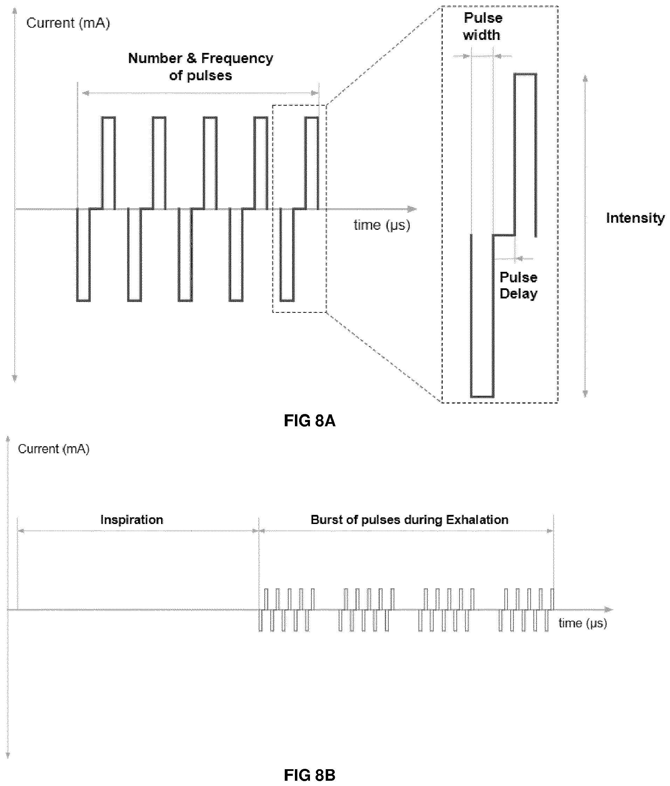

Moreover, an external charging case 13 and the connection of an internal application for smartphone 14 of the device 1 to an external cloud 15 configure a complete auricular neurostimulation system according to the invention, as shown in figure 6.
- Charging case 13: The auricular neurostimulation device 1 is stored when not used in a case 13 that charges its battery 10 wirelessly by electromagnetic induction. The device 1 discharges into this case 13 the data captured by the photoplethysmographic or biosensor 5 during stimulation when in use and sends them to a dedicated platform in the external cloud 15.
- Application for smartphone: The auricular neurostimulation device 1 has a smartphone application 14 that allows the patient or user to interact with the neurostimulation device 1, for example configuring certain stimulation parameters. The app 14 exchanges data with the dedicated platform in the cloud 15 to where the data captured by the photoplethysmographic or biosensor 5 during stimulation are sent.
- Stimulation protocols: The auricular neurostimulation device 1 implements a plurality of charge-controlled stimulation protocols. Preferably, these stimulation protocols are cathodic stimulation protocols. The charge is injected by applying an electrical voltage difference in the cymba electrode 2 with respect to the cavum electrode 3 that varies in real time depending on the impedance of the electrode-skin contact. The electrical voltage difference applied is a rectangular, biphasic, symmetrical wave with a delay between the first pulse and the second pulse (see Figure 8A). The first pulse of the waveform, or stimulating pulse, is used to elicit the desired physiological effect such as initiation of an action potential in the nerve endings, and the second pulse, or reversal pulse, is used to reverse electrochemical processes occurring during the stimulating pulse. The stimulation pulse is negative (cathodic stimulation) as it achieves faster depolarization of nerve endings than a positive pulse (anodic stimulation). It is estimated that the depolarization that occurs with anodic stimulation is roughly one-seventh to one-third that of the depolarization with cathodic stimulation (Daniel R. Merrill et al. 2004). Thus, cathodic stimulation requires less current to bring a nerve ending to threshold. The addition of a delay between the stimulation and reversal pulses also contributes to the reduction of the threshold to achieve the action potential of the nerve endings. However, the delay should not be too long to prevent the products of the Faradaic reactions caused by the stimulation pulse from accumulating to levels that could cause tissue damage. Delay values between 0 and 150µs are considered appropriate.

Stimulation protocols include pulse bursts as these improve the effectiveness of stimulation. The action potentials triggered at the sensory auricular vagus endings in response to continuous stimuli are less likely to influence systemic regulation or brain activity, rather than a rhythmic sequence of these impulses. This is because gradual natural sensory information is coded as the gradual temporal density of non-gradual impulses, likewise, coded as the instantaneous frequency of impulses. On the other hand, the brain with its very large number of neurons and its sophisticated processing is not likely to respond reasonably to a single or a few impulses but to a train of impulses. Stimulation protocols may include between 1 and 10 bursts per second.

The intensity of the electric current, the width of the pulses and their frequency are also variable in the stimulation protocols. The stimulation intensity can vary between 0 and 5mA as it has been proven experimentally that in this range is sufficient to produce an effective stimulation of nerve endings in a way that is comfortable for the user. The pulse width usually determines the type of fibers to be excited. That is, short pulses recruit easily excitable thick fibers only while elongated pulses recruit both thick and thin fibers. The ABVN is composed mainly of fibres Aβ, Aδ and C (Safi et al., 2016). The Aβ have diameters between 5 and 12µm and are associated with sensitive functions. The Aδ has diameters between 3 and 6µm and transmits localized pain, temperature and touch. Those of type C have diameters between 0,4 and 1,2µm and transmit diffuse pain and temperature. In stimulation, it is desirable to activate Aβ and not Aδ or C, so the stimulation pulse must be short. It has been proven that values between 50 and 250µs can be appropriate. Another important parameter in stimulation is the frequency or number of pulses per second, since depending on its value, one type of fiber or another is activated. The range of frequency variation in the stimulation protocols is between 1 and 30 Hz.

The different stimulation protocols can be conceptually grouped into three modalities:
- Protocols BEAT in which the patient's breathing is not taken into account.
- Protocols BFS that establish guidelines for the user to accommodate his breathing rhythm.
- Protocols EVANS in which the device automatically detects the user's inspiration and exhalation and stimulates only during exhalation.

BEAT type protocols apply pulse burst with variable parameters within the above ranges (see Figure 8A). The BSF and EVANS protocols also apply pulse bursts with variable parameters but only during user expiration (See Figure 8B). In the BSF the user adapts his exhalation to the moments of stimulation and in the EVANS the device detects the exhalation and synchronizes the stimulation with it.

The auricular neurostimulation device 1 of the invention is configured to detect when the user is exhaling thanks to the photoplethysmographic or biosensor 5. The duration of the stimulation sessions depends on the electrical charge (dosis) assigned to the session and the stimulation intensity selected by the user. Initially, each user is assigned an electrical charge depending on his/her profile, but based on the analysis of the data captured by the biosensor 5, the electrical charge to be applied can be customized. The stimulator keeps track of the applied electrical charge by stopping the stimulation when the set electric charge of the session has been reached. It is also possible to assign a maximum daily dose that the device will control not to be exceeded.

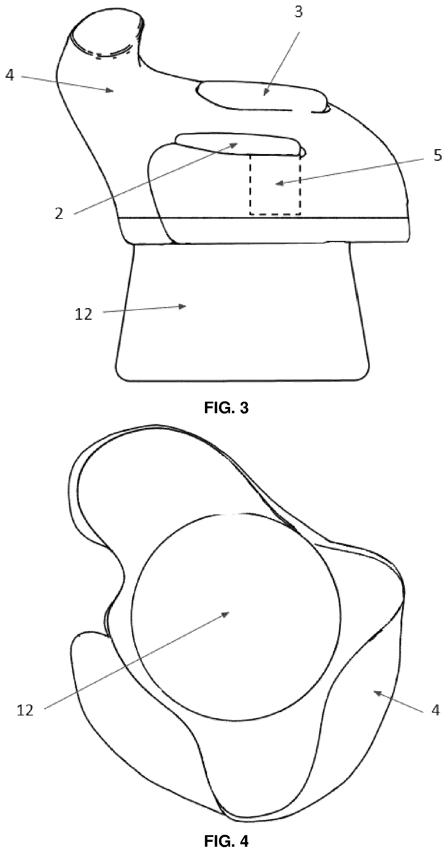

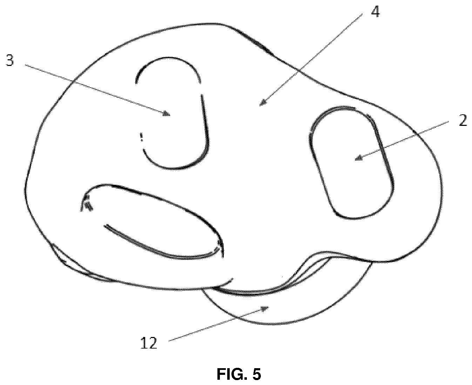

The auricular neurostimulation device 1 of the invention has been described according to a preferred embodiment, as represented in Figures 2-5: according to this embodiment, the electronic of the device 1 is placed in the conchae of the user's ear (ITE or In The Ear). However, a different possible embodiment of the device 1 of the invention would be to configure the device to be placed behind the ear (BTE) of the user, as represented in Figure 7. The components of the device are the same as in the preferred configuration (that represented in Figures 2-5) but having a different configuration allowing the device to be placed behind the ear of the user. This way, the electronic components of the device are arranged behind the user's ear and are not visible from outside. Moreover, the user is very comfortable with this BTE configuration.

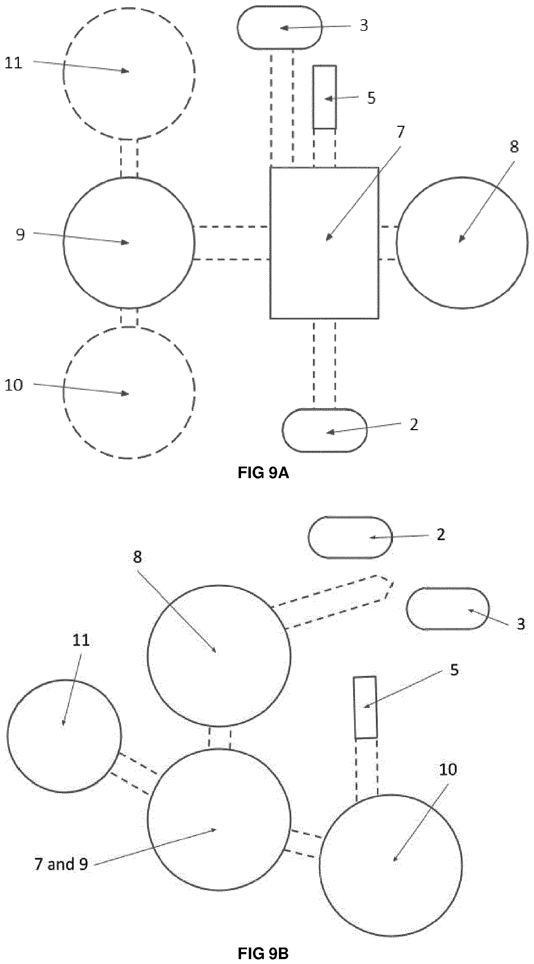

The electronic circuit 6 in the device of the invention is built on a Printed Circuit Board (PCB) that combines rigid parts with flexible parts, as shown in Figures 9A and 9B, each figure showing a different exemplary layout. The parts can be stacked to form an assembly that can be inserted into the faceplate 12, both in the ITE (In The Ear) configuration of Figures 2-5 and in the BTE (Behind The Ear) configuration of Figure 7. The electronic circuit 6 comprises the following elements:
- A central circuit 7 that controls all the functioning of the device, including the communication with the smartphone and the charger case, the generation of the stimulation patterns and the adjustment of the electrical voltage difference applied to the electrodes 2 and 3, according to the skin-electrode contact impedance.
- A voltage amplifier 8 that raises the voltage supplied by the battery to the level necessary to apply the electrical voltage difference to electrodes 2 and 3 required at any given time.
- A charger circuit 9 that takes advantage of the electric current generated in the coil 11.
- A battery 10 that is rechargeable with the current generated in coil 11.
- A coil 11 that receives the magnetic field created by a coil located in the charger case and generates electric current to recharge the battery.

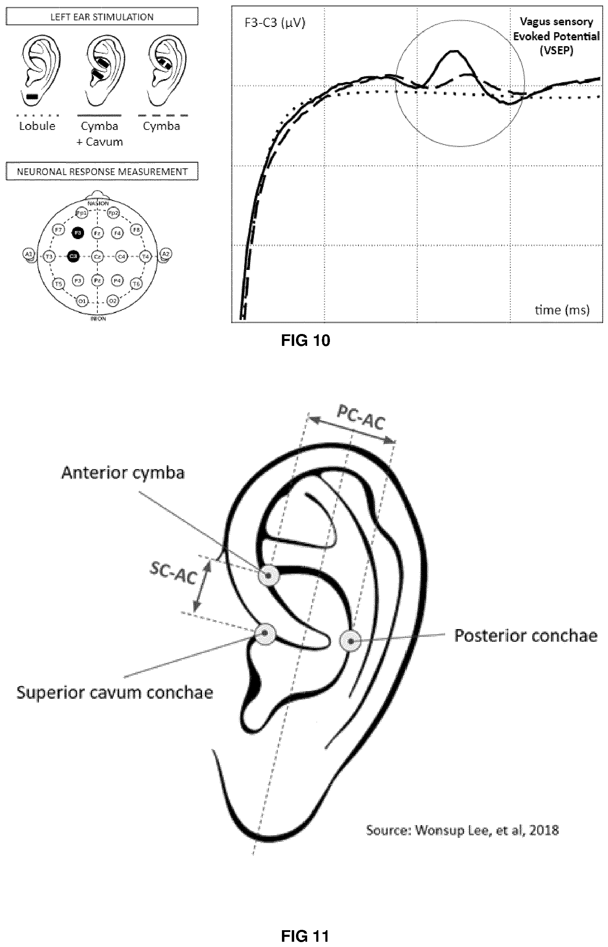

Figure 10 shows the Vagus sensory evoked potential (VSEP) induced by auricular stimulation applied a) in the lobe where there are no vagal nerve endings b) according to the electrode arrangement of the object of the present invention with two large electrodes, one in cymba as working electrode and one in cavum as counter electrode c) according to the electrode arrangement of Cerbomed's stimulator (corresponds with prior art device disclosed in EP3100764) with two small electrodes in cymba. Vagus sensory evoked potential (VSEP) via electrical ABVN stimulation and to measure it with EEG electrodes on the scalp as a far field potential has been demonstrated to be another way to evaluate the vagus nerve response (Fallgatter AJ et al, 2003,Lewine JD. et al., 2019). The graphs have been obtained by measuring neuronal electrical activity between points C3-F3 of the international 10-20 EEG measurement system. Neuronal activity is produced by the postsynaptic potentials generated in the nucleus of the solitary tract (NTS) in response to auricular electrical stimulation (See Figure 1B). The graphs shown have been obtained by averaging the neuronal response (vagal sensory evoked potential-VSEP) after the application of at least 50 electrical stimulation pulses. The amplitude of the VSEP is representative of the effectiveness of the stimulation. The results obtained in the measurement of 26 volunteers indicate that the stimulation performed as indicated in the present invention generates a vagal evoked potential with an amplitude 2.9 times greater than that generated by the stimulation of Cerbomed (prior art).

The auricular neurostimulation device 1 of the invention presents as explained below, several advantages with respect to other neurostimulation devices known in the state of the art, in particular relating to safety, effectiveness, comfort, usability and personalization:
Safety. The photoplethysmographic or biosensor 5 included in stimulator 1 makes it possible to anticipate a very low heart rate and stop the stimulation to prevent risky situations. On the other hand, circuit 6 keeps track of the daily electric charge introduced to the user, preventing it from exceeding a limit. It also adjusts in real time the electrical voltage difference applied to electrodes 2 and 3 according to the impedance of the contact of the electrodes with the skin, preventing the applied current from rising to dangerous limits if the impedance drops quickly, due to effects such as electroporation.
Effectiveness. The study carried out with vagus sensory evoked potentials concludes that stimulation with one electrode covering the whole cymba (electrode 2) and another in cavum (electrode 3) obtains a neuronal response associated with vagal activation 3.9 times greater than stimulation with two electrodes placed in the cymba. One reason for this is that with two electrodes in the cymba (100% vagal fibres) the whole surface of the cymba is not well used as there must be a minimum separation between them.

The surface of the cymba, as in general the anatomy of the whole ear, is very variable. In an anthropometric study carried out with 326 volunteers (Wonsup Lee, et al, 2018), the length was measured between superior cavum conchae and anterior cymba (SC-AC) and between the posterior conchae and anterior cymba (PC-AC) (see Figure 11).

The result was as follows:

| Length | Age group | | | |
|---|---|---|---|---|
| | 20s (n=133) | 30s (n=73) | 40s (n=55) | 50s (n=57) |
| Superior cavum conchae to anterior cymba (SC-AC) | 6,3 ± 1,5 mm | 6,3 ± 1,7 mm | 6,4 ± 1,3 mm | 6,6 ± 1,4 mm |
| Posterior conchae to anterior cymba (PC-AC) | 16,3 ± 2,0 mm | 15,8 ± 1,9 mm | 15,4 ± 1,6 mm | 14,9 ± 1,7 mm |

In terms of gender differences, the average length of SC-AC and PC-AC is greater for men than for women, 18% in the first case and 7% in the second.

The auricular neurostimulator device 1 of this invention is designed to maximize the stimulation in the cymba. When in this report the term '*large-surface electrode covering almost the whole cymba surface'* is used, it refers to an electrode 2 which occupies more than 75% of the cymba surface. Therefore, taking into account the human anthropometric standards and the figures indicated in the table above, electrode 2 has a surface between 25 mm² and 45 mm², depending on variables such as the age, sex and size, etc. of the user.

The electrode 3 is placed in the cavum conchae where 45% are vagal endings that are also stimulated.

Both electrode 2 and electrode 3 are placed on an earmold 4 customized to the anatomy of the user in order to ensure the best possible quality of contact between the electrodes and the area to be stimulated.

On the other hand, according to Merrill, et al, 2005, cathodic stimulation (the stimulation pulse is negative) is 3 to 7 times more effective than anodic stimulation and the addition of a delay between the stimulation pulse and the reversal pulse reduces tissue damage and improves the effectiveness of the stimulation.

The auricular neurostimulator device 1 of this invention implements a cathodic stimulation pattern on electrode 2 (cymba) and a delay between the stimulation pulse and the reversal pulse (see image 8A).

In addition, the device 1 can execute BSF or EVAN protocols, in which stimulation is synchronized with the user's exhalation, thus enhancing parasympathetic activation. During exhalation, the activation of arterial baroreceptors leads to the excitation of second-order neurons of the Nucleus Tractus Solitarii (NTS) that in turn increases premotor cardiovagal neuron firing rate. In addition, during inhalation, NTS receives inhibitory inputs from ventral respiratory nuclei in the medulla, reducing vagal outflow to the heart that could lead to respiratory sinus arrhythmia (RSA). As the dorsal medullary vagal system operates in tune with respiration, gating vagal afferent stimulation to the exhalation phase of respiration optimizes ABVN stimulation and its effects on cardiac vagal modulation.

Comfort. Transcutaneous electrical stimulation generates a throbbing sensation when the current density (amount of electrical current per unit area) is too high. The way to avoid this unpleasant sensation is to apply the electric current evenly over a large contact surface. To achieve this, device 1 of the invention uses large surface electrodes which are also placed in earmold 4 whose geometry is customized for each user. In this way, the contact zone between the electrodes and the stimulation zones is wide and of good quality so that the current can flow without concentrating excessively at any point.

Usability. The majority of auricular neurostimulators known in the state of the art consist of a large generator to which an accessory that applies an electrical voltage difference to some parts of the ear is connected by means of a cable. The volume and weight of the set limits its portability and consequently its availability for use. The auricular neurostimulation device 1 of the present invention, however, has been developed as a small, lightweight device that is comfortable to wear. Moreover, being configured as a wireless earbud or auricular, the user recognizes it as a familiar product so the adoption of use is very simple. In this regard, it is also important to highlight the technical characteristic that the miniaturized faceplate (12) incorporates inside it all the elements of an electronic circuit (6) built on a Printed Circuit Board (PCB) able to connect wirelessly with other devices or systems.

Personalization. The auricular neurostimulation device 1 of the invention includes two types of customizations: anatomical and therapeutic, as they will be explained in what follows.
- *Anatomical customization* consists of customizing the shape that is in contact with the user's ear. To achieve this, a sample is taken from the user's ear and then scanned. Another option is to scan the user's ear directly in 3D. The 3D geometry generated by both options is used to create a custom earmold 4 to which the faceplate 12 is added with the electronic circuit 6, and the stimulating device 1 is manufactured.
- *Therapeutic personalization* consists of personalizing the electrical charge (dosis) introduced by the stimulation and the possibility of stimulating it in a synchronized manner together with the user's breathing (EVANS/BFS protocols).

In addition, if the user integrates data from devices for continuous monitoring of cardiac activity such as watches, bracelets or rings, among others, the analysis of these data allows, for example, to know the pattern of evolution of stress of a user and define personalized stimulation treatments to prevent high peaks.

The auricular neurostimulation device 1 of the invention is a connected device, the connection of it being made through two pathways. On the one hand, it can connect wirelessly (e.g. via Bluetooth) to an application for a smartphone, which in turn is connected to a software in the cloud. On the other hand, the charging case 13 is connected to the software in the cloud, in order to be able to transmit the stimulator usage data, including those captured by the photoplethysmographic or biosensor 5.

In a broader way, as described earlier, the invention further relates to an auricular neurostimulation system comprising an auricular neurostimulation device 1 as described, an external charging case 13, and the connection of an internal application in the device to a cloud software for its correct connection and parametrization.

Although the present invention has been described with reference to preferred embodiments thereof, many modifications and alterations may be made by a person having ordinary skill in the art without departing from the scope of this invention, which is defined by the appended claims.

## Claims

1. Auricular neurostimulation device (1) wearable by a user and configured to stimulate the Auricular Branch of Vagus Nerve (ABVN) on the user's ear, wherein
the device (1) comprises at least two electrodes (2, 3) designed to be located in the cymba and in the cavity of the cavum conchae respectively;
the electrodes (2, 3) electrically stimulate the nerve ramifications of the cymba and the cavum conchae respectively;
**characterized in that**
the device (1) is configured as a wireless earbud comprising an earmold (4) and a miniaturized faceplate (12) wherein,
the electrodes (2, 3) are arranged on the earmold (4) which is customized to the user's ear anatomy such that the cymba electrode (2) is a large-surface electrode covering almost the whole cymba surface area of the user's ear.
a photoplethysmographic or biosensor (5) with a miniaturized configuration is arranged inside the earmold (4),
the miniaturized faceplate (12) incorporates inside it all the elements of an electronic circuit (6) built on a Printed Circuit Board (PCB) that combines rigid elements with flexible elements, both in an ITE (In The Ear) configuration or in an BTE (Behind The Ear) configuration.

2. Auricular neurostimulation device (1) according to claim 1 wherein the electrodes (2, 3) are made of graphene, biocompatible metals, non-toxic metals, conductive biocompatible inks for 3D printing or flexible conductive biocompatible polymers, and wherein the earmold (4) is made of a biocompatible material.

3. Auricular neurostimulation device (1) according to claim 2 wherein the biocompatible material the earmold (4) is made of is thermoelastic, so that it improves the fit with the user's ear as the earmold acquires body temperature.

4. Auricular neurostimulation device (1) according to claim 1,
wherein the photoplethysmographic or biosensor (5) measures the environment temperature and the user's temperature and to estimate the amount of hemoglobin and oxyhemoglobin circulating through the most superficial capillary vessels of the ear of the patient or user, these data being used to calculate the heart rate, the heart rate variability (HRV) and the oxygen saturation and to detect the breathing phase (exhalation or inhalation) of the user or patient;
and wherein the measurements made by the photoplethysmographic or biosensor (5) are used to determine which electrical charge is the most suitable for each user at any given time.

5. Auricular neurostimulation device (1) according to claim 1, wherein the electronic circuit (6) comprises the following elements:
• a central circuit (7) that controls all the functioning of the device (1),
• a voltage amplifier (8) that raises the voltage supplied by a battery (10),
• a charger circuit (9) that takes advantage of the electric current generated in a coil (11) that receives the magnetic field created by a further coil located in a charger case.
• a battery (10) that is rechargeable with the current generated in coil (11).

6. Auricular neurostimulation device (1) according to claims 1 - 5 , wherein the electronic circuit (6) is configured to:
• Generate stimulation patterns with variable duration, intensity, frequency of bursts and pulses, number of pulses per burst, pulse widths, and pulse delays, among others.
• Generate stimulation patterns synchronized with the exhalation of the user.
• Control the electrical charge applied in each stimulation and daily-accumulated charge.
• Exchange data with external devices through wireless connections.
• Exchange data with a charging case (13).
• Wireless charging of a battery (10) by electromagnetic induction with a charging case.

7. Auricular neurostimulation device (1) according to any of any of the previous claims, wherein the stimulation done by it, is synchronized with the exhalation-breathing phase of the user.

8. Auricular neurostimulation device (1) according to any of any of the previous claims, **characterized in that** it implements a plurality of stimulation protocols, where the intensity of the electric current, pulse width and repetition frequency of the pulses is variable;
and wherein the stimulation protocols are based on a waveform of rectangular, biphasic, symmetric and with a delay between the negative and positive pulse.

9. Auricular neurostimulation device (1) according to claims 7-8 wherein the stimulation protocol is any of the following types:
• a BEAT type protocol, consisting of the continuous application of bursts of pulses.
• a BFS (Breathing Focused on Stimulation) type protocol combining stimulation moments with standstill moments, so the user breathes in during the stop time and breathes out during the stimulation.
• an EVANS (Exhalation Vagus Auricular Nerve Stimulation) type protocol stimulating only during the exhalation of the user.

10. Auricular neurostimulation device (1) according to any of the previous claims wherein the electrical charge quantity applied to electrodes (2, 3) is personalized for each user, according to the therapeutic dose needed.

11. Auricular neurostimulation device (1) according to any of the previous claims wherein the electrical voltage difference applied to electrodes (2 and 3) is adjusted in real time to the impedance of the contact of the electrodes with the skin, in order to ensure that the intensity of stimulation is as established.

12. Auricular neurostimulation system comprising an auricular neurostimulation device (1) according to any of claims 1-11 and a charging case (13) where the device (1) is stored when not used in order to charge its battery (10) wirelessly by electromagnetic induction and where the device (1) discharges into this case (13) the data captured by the photoplethysmographic or biosensor (5) during stimulation and sends them to a dedicated platform in the cloud (15).

13. Auricular neurostimulation system comprising an auricular neurostimulation device (1) according to claim 12 wherein the neurostimulation device (1) is configurable via a smartphone application.

14. Method of configuration of an auricular neurostimulation system according to claim 13 comprising the following steps:
• Creation , via the smartphone application, by the user of a user account using the application for the smartphone or the web entering a series of personal data;
• Assignment, via the smartphone application, of an initial electrical charge value to apply in each stimulation session and a maximum daily electrical charge, depending on the user's profile and on the basis of statistical studies;
• Detection of the user logging into the application;
• Connection to the auricular neurostimulation device (1) to match a serial number of the auricular neurostimulation device (1) to the user account, so the device (1) is associated to the user;
• Prompting the user to set his/her 'perception and pain thresholds';
• based on both thresholds establishment of a range in which the stimulation intensity must be placed, so that the stimulation is effective but also comfortable;
• Detection of a selection of a stimulation protocol by the user; and
• Configure the auricular neurostimulation device (1) in accordance with the range of stimulation intensity and the stimulation protocol

15. Method of configuration of an auricular neurostimulation system according to claim 13 comprising the following steps after a stimulation session:
- Reception, from the auricular neurostimulation device (1) and at the charging case (13) or the smartphone application (14), session data including readings stored by the photoplethysmographic or biosensor (5);
- Transmission of the data from the stimulation session to a cloud platform for storage thereat;
- Reception of a result of an algorithm that analyses data stored at the cloud platform to determine an optimized dose of electrical charge required by the user;
- Enablement of the user to modify the optimized dose of electrical charge; and
- Reception of a notification recommending stimulation sessions that prevent stress peaks based on an analysis of the data stored at the cloud platform obtained from other devices associated with the user that continuously monitor cardiac activity of the user.

## Patentansprüche

1. Aurikuläre Neurostimulationsvorrichtung (1), die von einem Benutzer getragen werden kann und dazu ausgebildet ist, den Ramus auricularis nervi vagi (ABVN) am Ohr des Benutzers zu stimulieren, wobei
die Vorrichtung (1) mindestens zwei Elektroden (2, 3) umfasst, die dazu bestimmt sind, jeweils in der Cymba und in der Vertiefung des Cavum conchae angebracht zu werden;
die Elektroden (2, 3) die Nervenverzweigungen der Cymba bzw. des Cavum conchae elektrisch stimulieren; **dadurch gekennzeichnet, dass**
die Vorrichtung (1) als kabelloser Ohrhörer ausgebildet ist, der ein Ohrpassstück (4) und eine miniaturisierte Frontplatte (12) umfasst, wobei
die Elektroden (2, 3) auf dem Ohrpassstück (4) angeordnet sind, das an die Ohranatomie des Benutzers angepasst ist, so dass die Cymba-Elektrode (2) eine großflächige Elektrode ist, die nahezu die gesamte Cymba-Oberfläche des Ohres des Benutzers abdeckt,
innerhalb des Ohrpassstücks (4) ein Photoplethysmographie- oder Biosensor (5) mit miniaturisierter Konfiguration angeordnet ist,
wobei die miniaturisierte Frontplatte (12) alle Elemente einer elektronischen Schaltung (6) enthält, die auf einer Leiterplatte (PCB) aufgebaut ist, die starre Elemente mit flexiblen Elementen kombiniert, sowohl in einer ITE- ("In The Ear", im Ohr) Konfiguration als auch in einem BTH-("Behind The Ear", Hinter dem Ohr) Konfiguration.

2. Aurikuläre Neurostimulationsvorrichtung (1) nach Anspruch 1, wobei die Elektroden (2, 3) aus Graphen, biokompatiblen Metallen, ungiftigen Metallen, leitfähigen biokompatiblen Tinten für den 3D-Druck, oder flexiblen leitfähigen biokompatiblen Polymeren bestehen, und wobei das Ohrpassstück (4) aus einem biokompatiblen Material besteht.

3. Aurikuläre Neurostimulationsvorrichtung (1) nach Anspruch 2, wobei das biokompatible Material, aus dem das Ohrpassstück (4) besteht, thermoelastisch ist, so dass es den Sitz am Ohr des Benutzers verbessert, wenn das Ohrpassstück Körpertemperatur annimmt.

4. Aurikuläre Neurostimulationsvorrichtung (1) nach Anspruch 1,
wobei der Photoplethysmographie- oder Biosensor (5) die Umgebungstemperatur und die Temperatur des Benutzers misst und die Menge an Hämoglobin und Oxyhämoglobin schätzt, die durch die oberflächlichsten Kapillargefäße des Ohrs des Patienten oder Benutzers zirkuliert, wobei diese Daten dazu verwendet werden, die Herzfrequenz, die Herzfrequenzvariabilität (HRV) und die Sauerstoffsättigung zu berechnen sowie die Atemphase (Ausatmung oder Einatmung) des Benutzers oder Patienten zu erkennen;
und wobei die Messungen des Photoplethysmographie- oder Biosensors (5) verwendet werden, um zu bestimmen, welche elektrische Ladung für jeden Benutzer zu einem bestimmten Zeitpunkt am besten geeignet ist.

5. Aurikuläre Neurostimulationsvorrichtung (1) nach Anspruch 1, wobei die elektronische Schaltung (6) folgende Elemente umfasst:
• einen zentralen Schaltkreis (7), der alle Funktionen der Vorrichtung (1) steuert,
• einen Spannungsverstärker (8), der die von einer Batterie (10) gelieferte Spannung erhöht,
• eine Ladeschaltung (9), die den elektrischen Strom nutzt, der in einer Spule (11) erzeugt wird, die das Magnetfeld empfängt, das von einer weiteren Spule in einem Ladecase erzeugt wird.
• eine Batterie (10), die mit dem in der Spule (11) erzeugten Strom wieder aufgeladen werden kann.

6. Aurikuläre Neurostimulationsvorrichtung (1) nach den Ansprüchen 1-5, wobei die elektronische Schaltung (6) ausgebildet ist zum:
• Erzeugen von Stimulationsmustern mit, unter anderem, variabler Dauer, Intensität, Häufigkeit von Bursts und Pulsen, Anzahl der Pulse pro Burst, Pulsbreiten und Pulsverzögerungen,
• Erzeugen von Stimulationsmustern, die mit der Ausatmung des Benutzers synchronisiert sind,
• Steuern der elektrischen Ladung, die bei jeder Stimulation angelegt wird, und einer täglich akkumulierten Ladung,
• Austauschen von Daten mit externen Vorrichtungen über drahtlose Verbindungen,
• Austauschen von Daten mit einem Ladecase (13),
• kabellosen Laden eines Akkus (10) durch elektromagnetische Induktion mit einem Ladecase.

7. Aurikuläre Neurostimulationsvorrichtung (1) nach einem der vorhergehenden Ansprüche, wobei die damit durchgeführte Stimulation mit der Ausatmungs-Atmungsphase des Benutzers synchronisiert ist.

8. Aurikuläre Neurostimulationsvorrichtung (1) nach einem der vorhergehenden Ansprüche, **dadurch gekennzeichnet, dass** sie eine Mehrzahl von Stimulationsprotokollen implementiert, wobei die Intensität des elektrischen Stroms, die Impulsbreite und die Wiederholungsfrequenz der Impulse variabel sind; und wobei die Stimulationsprotokolle auf einer rechteckigen, zweiphasigen, symmetrischen Wellenform mit einer Verzögerung zwischen dem negativen und positiven Impuls basieren.

9. Aurikuläre Neurostimulationsvorrichtung (1) nach den Ansprüchen 7-8, wobei das Stimulationsprotokoll von einem der folgenden Typen ist:
• ein BEAT-Protokoll, das aus der kontinuierlichen Anwendung von Impulsstößen besteht,
• ein BFS- ("Breathing Focused on Stimulation", auf Stimulation fokussierte Atmung) Protokoll, das Stimulationsmomente mit Stillstandsmomenten kombiniert, so dass der Benutzer während der Stoppzeit einatmet und während der Stimulation ausatmet,
• ein EVANS- (Vagus Auricular Nerv-Stimulation-Ausatmung) Protokoll, das nur während der Ausatmung des Benutzers stimuliert.

10. Aurikuläre Neurostimulationsvorrichtung (1) nach einem der vorherigen Ansprüche, wobei die an die Elektroden (2, 3) angelegte elektrische Ladungsmenge für jeden Benutzer entsprechend der benötigten therapeutischen Dosis individuell angepasst wird.

11. Aurikuläre Neurostimulationsvorrichtung (1) nach einem der vorherigen Ansprüche, wobei die an die Elektroden (2 und 3) angelegte elektrische Spannungsdifferenz in Echtzeit an die Impedanz des Kontakts der Elektroden mit der Haut angepasst wird, um sicherzustellen, dass die Intensität der Stimulation wie festgelegt ist.

12. Aurikuläres Neurostimulationssystem, umfassend eine aurikuläre Neurostimulationsvorrichtung (1) nach einem der Ansprüche 1-10,
und ein Ladecase (13), in dem die Vorrichtung (1) aufbewahrt wird, wenn sie nicht verwendet wird, um ihre Batterie (10) drahtlos durch elektromagnetische Induktion aufzuladen, und wobei die Vorrichtung (1) die von dem Photoplethysmographie- oder Biosensor (5) während der Stimulation erfassten Daten in dieses Case (13) entlädt und an eine spezielle Plattform in der Cloud (15) sendet.

13. Aurikuläres Neurostimulationssystem, umfassend eine aurikuläre Neurostimulationsvorrichtung (1) nach Anspruch 12, wobei die Neurostimulationsvorrichtung (1) über eine Smartphone-Anwendung konfigurierbar ist.

14. Verfahren zur Konfiguration eines aurikulären Neurostimulationssystems nach Anspruch 13, umfassend die folgenden Schritte:
• Erstellen, über die Smartphone-Anwendung, eines Benutzerkontos durch den Benutzer unter Verwendung der Anwendung für das Smartphone oder das Internet, indem eine Reihe personenbezogener Daten eingegeben werden;
• Zuweisen, über die Smartphone-Anwendung, eines anfänglichen elektrischen Ladungswerts für jede Stimulationssitzung und einer maximalen täglichen elektrischen Ladung in Abhängigkeit von dem Profil des Benutzers und basierend auf statistische Studien;
• Erkennen der Benutzeranmeldung bei der Anwendung;
• Verbinden mit der aurikulären Neurostimulationsvorrichtung (1), um eine Seriennummer der aurikulären Neurostimulationsvorrichtung (1) mit dem Benutzerkonto abzugleichen, so dass die Vorrichtung (1) dem Benutzer zugeordnet wird;
• Auffordern des Benutzers, seine ,Wahrnehmungs- und Schmerzschwellenwerte' einzustellen;
• basierend auf beiden Schwellenwerten, Festlegen eines Bereichs, in dem die Stimulationsintensität liegen muss, damit die Stimulation effektiv, aber auch angenehm ist.
• Erkennen einer Auswahl eines Stimulationsprotokolls durch den Benutzer; und
• Konfigurieren der aurikulären Neurostimulationsvorrichtung (1) entsprechend dem Stimulationsintensitätsbereich und dem Stimulationsprotokoll

15. Verfahren zur Konfiguration eines aurikulären Neurostimulationssystems nach Anspruch 13, umfassend die folgenden Schritte nach einer Stimulationssitzung:
• Empfangen, von der aurikulären Neurostimulationsvorrichtung (1) und bei dem Ladecase (13) oder der Smartphone-Anwendung (14), von Sitzungsdaten, die von dem Photoplethysmographie- oder Biosensor (5) gespeicherte Messwerte aufweist;
• Übertragung der Daten aus der Stimulationssitzung an eine Cloud-Plattform zur dortigen Speicherung;
• Empfangen eines Ergebnisses eines Algorithmus, der auf der Cloud-Plattform gespeicherte Daten analysiert, um eine optimierte Dosis elektrischer Ladung zu bestimmen, die der Benutzer benötigt;
• Ermöglichen, dass der Benutzers die optimierte Dosis elektrischer Ladung ändert; und
• Empfangen einer Benachrichtigung, die Stimulationssitzungen empfiehlt, die Stressspitzen verhindern, basierend auf einer Analyse der auf der Cloud-Plattform gespeicherten Daten, die von anderen dem Benutzer zugeordneten Vorrichtungen stammen, die die Herzaktivität des Benutzers kontinuierlich überwachen.

## Revendications

1. Dispositif (1) de neurostimulation auriculaire portable par un utilisateur et configuré pour stimuler le rameau auriculaire du nerf vague (ABVN) sur l'oreille de l'utilisateur,
le dispositif (1) comprenant au moins deux électrodes (2, 3) conçues pour être situées dans la cymba et dans la cavité de la conque respectivement ;
les électrodes (2, 3) stimulant électriquement les ramifications de nerf de la cymba et du conque respectivement ;
**caractérisé en ce que**
le dispositif (1) est configuré comme une oreillette sans fil comprenant un moule auriculaire (4) et une plaque frontale miniaturisée (12),
les électrodes (2, 3) étant agencées sur le moule auriculaire (4) qui est personnalisé pour l'anatomie d'oreille de l'utilisateur de telle sorte que l'électrode (2) de cymba est une électrode à grande surface couvrant presque toute la zone de surface de cymba de l'oreille de l'utilisateur,
un capteur photopléthysmographique ou biocapteur (5) avec une configuration miniaturisée étant agencé à l'intérieur du moule auriculaire (4),
la plaque frontale miniaturisée (12) intégrant à l'intérieur de celle-ci tous les éléments d'un circuit électronique (6) fabriqué sur une carte de circuit imprimé (PCB) qui combine des éléments rigides avec des éléments souples, à la fois dans une configuration ITE (dans l'oreille) et dans une configuration BTE (derrière l'oreille).

2. Dispositif (1) de neurostimulation auriculaire selon la revendication 1, les électrodes (2, 3) étant faites de graphène, de métaux biocompatibles, de métaux non toxiques, d'encres biocompatibles conductrices pour impression 3D ou de polymères biocompatibles conducteurs souples, et le moule auriculaire (4) étant fait d'un matériau biocompatible.

3. Dispositif (1) de neurostimulation auriculaire selon la revendication 2, le matériau biocompatible du moule auriculaire (4) étant fait d'un thermoélastique, de sorte qu'il améliore l'ajustement à l'oreille de l'utilisateur dès que le moule auriculaire acquière la température du corps.

4. Dispositif (1) de neurostimulation auriculaire selon la revendication 1,
le capteur photopléthysmographique ou biocapteur (5) mesurant la température ambiante et la température de l'utilisateur et étant destiné à estimer la quantité d'hémoglobine et d'oxyhémoglobine circulant à travers les vaisseaux capillaires les plus en surface de l'oreille du patient ou utilisateur, ces données étant utilisées pour calculer la fréquence cardiaque, la variabilité de fréquence cardiaque (HRV) et la saturation en oxygène et pour détecter la phase de respiration (expiration ou inspiration) de l'utilisateur ou patient ;
et les mesures réalisées par le capteur photopléthysmographique ou biocapteur (5) étant utilisées pour déterminer quelle charge électrique est la plus appropriée pour chaque utilisateur à un instant donné quelconque.

5. Dispositif (1) de neurostimulation auriculaire selon la revendication 1, le circuit électronique (6) comprenant les éléments suivants :
• un circuit central (7) qui commande tout le fonctionnement du dispositif (1),
• un amplificateur de tension (8) qui augmente la tension fournie par une batterie (10),
• un circuit de charge (9) qui tire avantage du courant électrique généré dans une bobine (11) qui reçoit le champ magnétique créé avec une autre bobine située dans un boitier de chargeur,
• une batterie (10) rechargeable avec le courant généré dans la bobine (11).

6. Dispositif (1) de neurostimulation auriculaire selon les revendications 1 à 5, le circuit électronique (6) étant configuré pour :
• générer des motifs de stimulation avec une durée, une intensité, une fréquence de salves et d'impulsions, un nombre d'impulsions par salve, des largeurs d'impulsions et des retards d'impulsion, parmi d'autres, différents,
• générer des motifs de stimulation synchronisés avec l'expiration de l'utilisateur,
• commander la charge électrique appliquée dans chaque stimulation et la charge accumulée quotidiennement,
• échanger des données avec des dispositifs externes via des connexions sans fil,
• échanger des données avec un boitier de charge (13),
• charger sans fil une batterie (10) par induction électromagnétique avec un boitier de charge.

7. Dispositif (1) de neurostimulation auriculaire selon l'une quelconque des revendications précédentes, la stimulation réalisée par celui-ci étant synchronisée avec la phase de respiration d'expiration de l'utilisateur.

8. Dispositif (1) de neurostimulation auriculaire selon l'une quelconque des revendications précédentes, **caractérisé en ce qu'**il met en œuvre une pluralité de protocoles de stimulation, où l'intensité du courant électrique, la largeur d'impulsions et la fréquence de répétition des impulsions sont variables ;
et les protocoles de stimulation étant basés sur une forme d'onde rectangulaire, biphasique, symétrique et avec un retard entre l'impulsion négative et l'impulsion positive.

9. Dispositif (1) de neurostimulation auriculaire selon les revendications 7 et 8, le protocole de stimulation étant l'un quelconque des types suivants :
• un protocole de type BEAT, consistant en l'application en continu de salves d'impulsions,
• un protocole de type BFS (respiration focalisée sur la stimulation) associant des instants de stimulation à des instants d'arrêt, de sorte que l'utilisateur inspire pendant le temps d'arrêt et expire pendant la stimulation,
• un protocole de type EVANS (stimulation du rameau auriculaire du nerf vague à l'expiration) stimulant uniquement pendant l'expiration de l'utilisateur.

10. Dispositif (1) de neurostimulation auriculaire selon l'une quelconque des revendications précédentes, la quantité de charge électrique appliquée à des électrodes (2, 3) étant personnalisée pour chaque utilisateur, en fonction de la dose thérapeutique nécessaire.

11. Dispositif (1) de neurostimulation auriculaire selon l'une quelconque des revendications précédentes, la différence de tension électrique appliquée à des électrodes (2 et 3) étant ajustée en temps réel à l'impédance du contact des électrodes avec la peau, afin de garantir que l'intensité de la stimulation est telle qu'établie.

12. Système de neurostimulation auriculaire comprenant un dispositif (1) de neurostimulation auriculaire selon l'une quelconque des revendications 1 à 10 et un boitier de charge (13) où est stocké le dispositif (1) lorsqu'il n'est pas utilisé afin de charger sa batterie (10) sans fil par induction électromagnétique et où le dispositif (1) décharge dans ce boitier (13) les données capturées par le capteur photopléthysmographique ou biocapteur (5) pendant la stimulation et les envoie à une plateforme dédiée dans l'infonuage (15).

13. Système de neurostimulation auriculaire comprenant un dispositif (1) de neurostimulation auriculaire selon la revendication 12, le dispositif (1) de neurostimulation pouvant être configuré via une application de téléphone intelligent.

14. Procédé de configuration d'un système de neurostimulation auriculaire selon la revendication 13, comprenant les étapes suivantes :
• création, via l'application de téléphone intelligent, par l'utilisateur d'un compte utilisateur utilisant l'application pour le téléphone intelligent ou le web en saisissant une série de données personnelles ;
• attribution, via l'application de téléphone intelligent, d'une valeur de charge électrique initiale à appliquer dans chaque session de stimulation et d'une charge électrique quotidienne maximale, en fonction du profil de l'utilisateur et sur la base d'études statistiques ;
• détection de la connexion de l'utilisateur à l'application ;
• connexion au dispositif (1) de neurostimulation auriculaire pour faire correspondre un numéro de série du dispositif (1) de neurostimulation auriculaire au compte utilisateur, de sorte que le dispositif (1) est associé à l'utilisateur ;
• invitation de l'utilisateur à définir ses « seuils de perception et de douleur » ;
• sur la base des deux seuils, établissement d'une plage dans laquelle l'intensité de stimulation doit se situer, de sorte que la stimulation est efficace mais également confortable ;
• détection d'une sélection d'un protocole de stimulation par l'utilisateur ; et
• configuration du dispositif (1) de neurostimulation auriculaire en fonction de la plage de l'intensité de stimulation et du protocole de stimulation.

15. Procédé de configuration d'un système de neurostimulation auriculaire selon la revendication 13 comprenant les étapes suivantes après une session de stimulation :
• réception, en provenance du dispositif (1) de neurostimulation auriculaire et au niveau du boitier de charge (13) ou de l'application de téléphone intelligent (14), de données de session comportant des lectures stockées par le capteur photopléthysmographique ou biocapteur (5) ;
• transmission des données de la session de stimulation à une plateforme d'infonuage pour un stockage au niveau de celui-ci ;
• réception d'un résultat d'un algorithme qui analyse des données stockées au niveau de la plateforme d'infonuage pour déterminer une dose optimisée de charge électrique requise par l'utilisateur ;
• permission à l'utilisateur de modifier la dose optimisée de charge électrique ; et
• réception d'une notification recommandant des sessions de stimulation qui préviennent des pics de stress sur la base d'une analyse des données stockées au niveau de la plateforme d'infonuage obtenues en provenance d'autres dispositifs associés à l'utilisateur qui surveillent en continu l'activité cardiaque de l'utilisateur.
